(19) Europäisches Patentamt
European Patent Office
Office européen des brevets

(11) Numéro de publication : 0 242 265 B1

(12) # FASCICULE DE BREVET EUROPEEN

(45) Date de publication du fascicule du brevet : **29.05.91 Bulletin 91/22**

(51) Int. Cl.[5] : **C07D 281/10**, A61K 31/55

(21) Numéro de dépôt : **87400750.3**

(22) Date de dépôt : **06.04.87**

(54) **Dérivés de Benzothiazépine-1,5, leur préparation et leur application en thérapeutique.**

Le dossier contient des informations techniques présentées postérieurement au dépôt de la demande et ne figurant pas dans le présent fascicule.

(30) Priorité : **15.04.86 FR 8605346**

(43) Date de publication de la demande : **21.10.87 Bulletin 87/43**

(45) Mention de la délivrance du brevet : **29.05.91 Bulletin 91/22**

(84) Etats contractants désignés : **AT BE CH DE ES FR GB GR IT LI LU NL SE**

(56) Documents cités :
**US-A- 3 341 519**
**US-A- 3 562 257**

(73) Titulaire : **SYNTHELABO**
**58 rue de la Glacière**
**F-75013 Paris (FR)**

(72) Inventeur : **Muller, Jean-Claude**
**4, Avenue de l'Espérance**
**F-91390 Morsang sur Orge (FR)**
Inventeur : **Dumas, André**
**9, Clos Madeleine**
**F-91120 Palaiseau (FR)**

(74) Mandataire : **Ludwig, Jacques et al**
**SYNTHELABO, Service Brevets, 22, avenue Galilée, B.P. 72**
**F-92352 Le Plessis Robinson Cédex (FR)**

**EP 0 242 265 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention a pour objet des dérivés de benzothiazépine-1,5, leur préparation et leur application en thérapeutique.

Les composés de l'invention répondent à la formule générale (I) donnée dans le schéma 1 de la page suivante, formule dans laquelle

R représente l'hydrogène ou un groupe alkyle en $C_1$-$C_4$ linéaire ou ramifié, et

$R_1$ et $R_2$, séparément, représentent chacun un groupe alkyle en $C_1$-$C_4$ linéaire ou ramifié, ou bien

$R_1$ et $R_2$, ensemble, représentent un groupe tétraméthylène ou pentaméthylène.

La molécule de formule (I) comprend plusieurs centres chiraux, de sorte que les composés peuvent se présenter sous forme d'énantiomères purs, de diastéréoisomères et de leurs mélanges. Les divers isomères optiques font bien entendu partie de l'invention.

Enfin les composés (I) peuvent exister à l'état de bases libres ou de sels d'addition à des acides.

L'invention englobe également ces diverses formes. Des composés de structures proches de celle des composés de l'invention, et utilisables dans le même domaine thérapeutique, sont décrits dans les brevets US-3341519 et US-3562257.

Conformément à l'invention on peut préparer les composés (I) selon le schéma 1 ci-après.

On traite d'abord une benzothiazépinone de formule (II) par l'hydrure de sodium, dans un solvant tel que le diméthylsulfoxyde puis, dans le même récipient, on fait réagir le composé obtenu avec l'épichlorhydrine (Z = Cl) ou avec le tosylate de glycidyle (Z = tosyloxy), puis avec une amine de formule $HNR_1R_2$, $R_1$ et $R_2$ étant tels que définis ci-dessus. L'intermédiaire de formule (III) ainsi obtenu est un mélange de deux diastéréoisomères A et B, si le composé de départ (II) et le dérivé glycidylique (épichlorhydrine ou tosylate de glycidyle) sont sous forme de racémates. Deux diastéréoisomères mélangés peuvent être séparés par cristallisation fractionnée. Il va de soi que l'on peut préparer un seul diastéréoisomère ou un seul énantiomère selon que l'on utilise un composé de départ (II) et/ou un dérivé glycidylique sous forme d'énantiomère pur.

La dernière étape du procédé consiste en une acylation, par un halogénure d'acyle RCOX, R étant tel que défini ci-dessus, dans un solvant tel que le dichlorométhane, et en présence d'une base telle que la pyridine ou la diméthylamino-4 pyridine, pour fixer l'acide chlorhydrique libéré.

Schéma 1

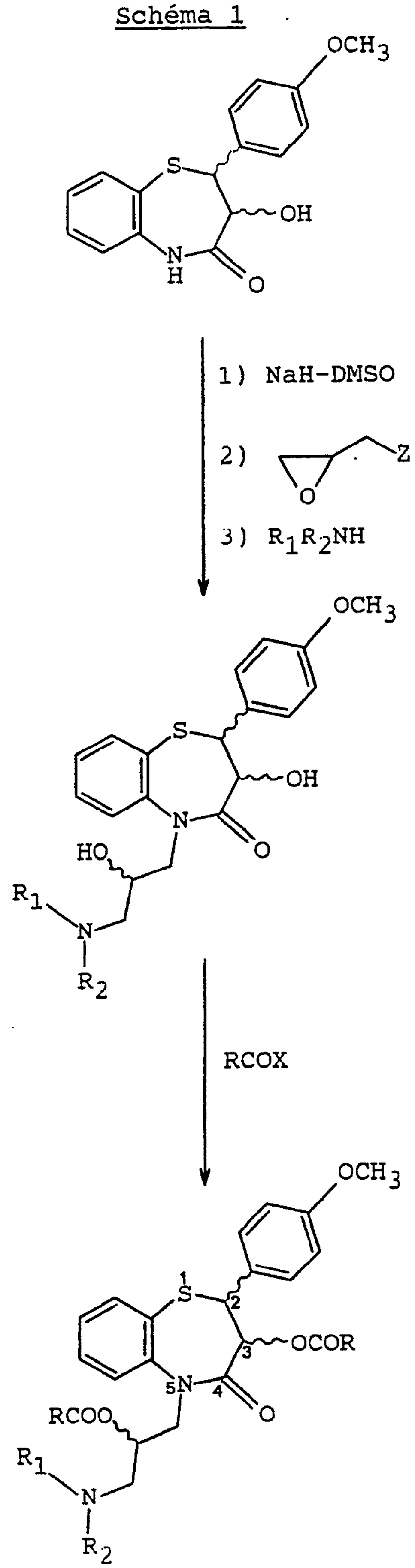


(II)

(III)

(I)

Schéma 2

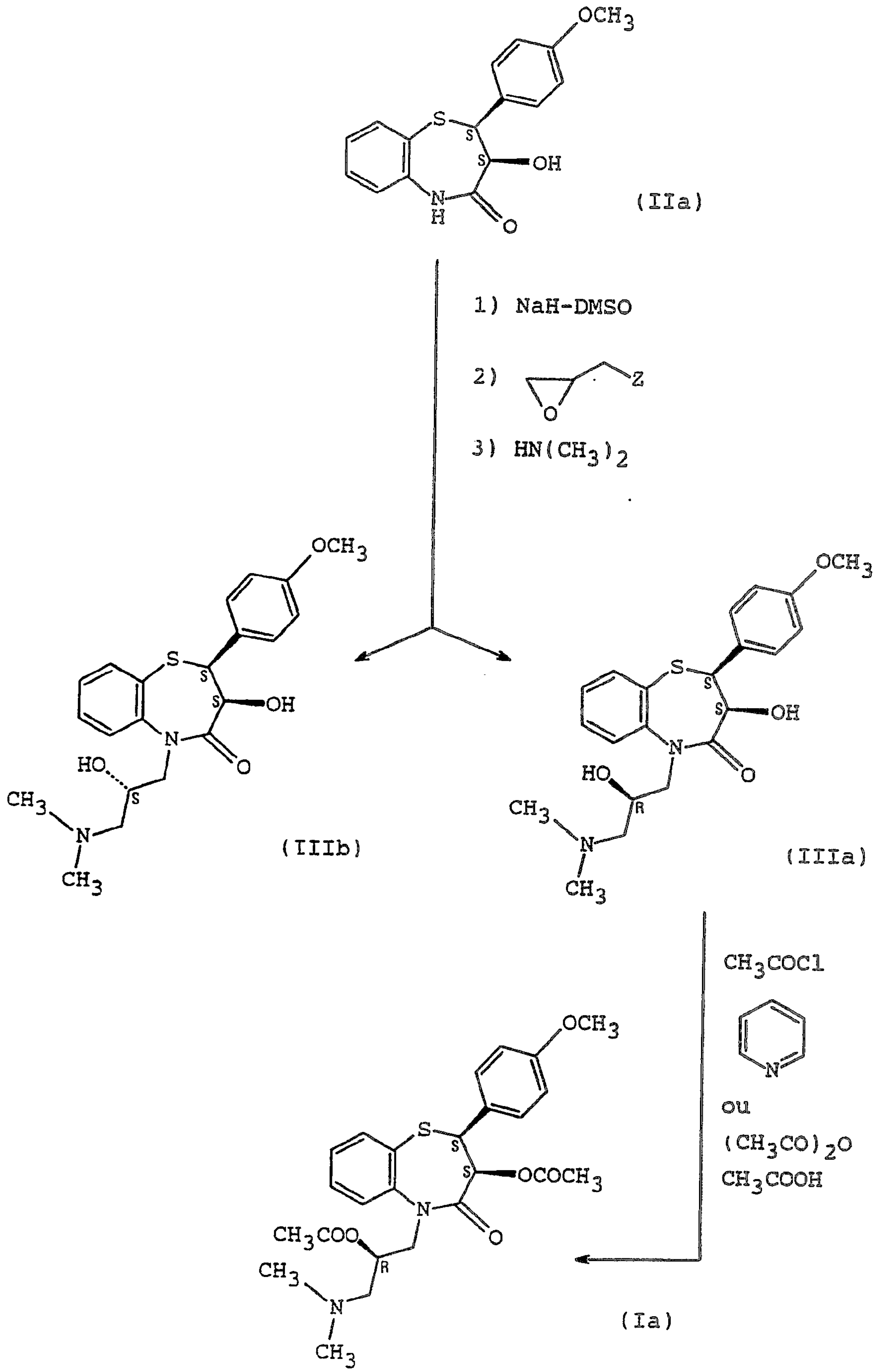


(IIa)

(IIIb)

(IIIa)

(Ia)

Le schéma 2 de la page précédente illustre en particulier la préparation, stréospécifique, du composé préféré de l'invention, en l'espèce la (+)cis 2S,3S-(méthoxy-4 phényl)-2 acétyloxy-3 (diméthylamino-3R acétyloxy-2 propyl)-5 dihydro-2,3 5H-benzothiazépine-1,5 one-4. Les formules (Ia), (IIa), (IIIa) et (IIIb) correspondent aux formules (I), (II) et (III) dans lesquelles R, $R_1$ et $R_2$ représentent chacun un groupe méthyle.
Ce schéma 2 illustre également une variante de l'étape finale d'acylation, en l'espèce une acétylation au moyen d'anhydride acétique et d'acide acétique.

Les exemples qui vont suivre illustrent en détail la préparation de quelques composés selon l'invention. Les micro-analyses et les spectres IR et RMN ont confirmé les structures des produits obtenus.

Exemple 1

Dans un réacteur de 500 ml sous atmosphère inerte, on introduit 3 g (0,062 mole) d'hydrure de sodium à 50% et 150 ml de diméthylsulfoxyde. On agite le mélange à 70°C durant 1 h puis après avoir refroidi à 20°C on ajoute 18,7 g (0,062 mole) de (±)cis (méthoxy-4 phényl)-2 hydroxy-3 dihydro-2,3 5H-benzothiazépine-1,5 one-4 dissoute dans 60 ml de diméthylsulfoxyde. On maintient l'agitation à 30°C durant 1 h puis on refroidit à 15°C et on additionne 19,6 ml (0,248 mole) d'épichlorhydrine. Après 2 h d'agitation à 20°C, on ajoute 41 g de diméthylamine et on chauffe le milieu réactionnel à 60°C durant 3 h.
On jette le milieu réactionnel brut sur une solution de bicarbonate glacée ; on filtre le précipité obtenu et on le lave copieusement à l'eau. On reprend le solide dans du dichlorométhane, le lave à l'eau et le sèche. Après évaporation, on isole 17,5 g d'un mélange brut qu'on traite par une solution d'acide chlorhydrique dans l'éther et l'éthanol. On isole 9,7 g d'un mélange de diastéréoisomères. Par cristallisations successives, on obtient sélectivement l'un ou l'autre des diastéréoisomères :

Chlorhydrate de (±)cis (méthoxy-4 phényl)-2 hydroxy-3 (diméthylamino-3 hydroxy-2 propyl)-5 dihydro-2,3 5H-benzothiazépine-1,5 one-4
Diastéréoisomère A F = 213-217°C
Diastéréoisomère B F = 194-195°C.

On dissout 4,02 g (0,01 mole) de mélange de diastéréoisomères dans 150 ml de dichlorométhane et 15 ml de pyridine. On refroidit le mélange réactionnel dans la glace puis le traite par 3 ml de chlorure d'acétyle. On maintient l'agitation durant une nuit puis on évapore les solvants sous vide. On dissout le produit brut dans du dichlorométhane puis on le lave avec une solution saturée de bicarbonate de sodium. Après séchage et évaporation du solvant on traite le résidu par l'éthanol chlorhydrique.
Après cristallisation on isole 3,94 g de chlorhydrate de (±)cis (méthoxy-4 phényl)-2 acétyloxy-3 (diméthylamino-3 acétyloxy-2 propyl)-5 dihydro-2,3 5H-benzothiazépine-1,5 one-4 (mélange de diastéréoisomères).
F = 232°C.

On dissout 0,8 g (1,82 mole) du diastéréoisomère B dans une solution de 40 ml de dichlorométhane contenant 3 ml de pyridine et on les traite à 0°C par 0,6 ml de chlorure d'acétyle. On laisse le mélange au repos pendant une nuit puis on évapore le solvant. On reprend le résidu brut dans du dichlorométhane, on le lave par une solution de bicarbonate de sodium. Après séchage et évaporation du solvant, on traite le résidu par l'éthanol chlorhydrique.
On isole quantitativement le chlorhydrate de (±)cis (méthoxy-4 phényl)-2 acétyloxy-3 (diméthylamino-3 acétyloxy-2 propyl)-5 dihydro-2,3 5H-benzothiazépine-1,5 one-4.
Diastéréoisomère B F = 145°C.
On dissout 1,4 g (3,19 mole) du diastéréoisomère A dans 70 ml de dichlorométhane et 5 ml de pyridine et on les traite à 0°C par 1 ml de chlorure d'acétyle. On maintient l'agitation durant une nuit puis on évapore les solvants sous vide. On reprend le mélange brut dans du dichlorométhane lavé par une solution saturée de bicarbonate de sodium. Après séchage et évaporation du solvant, on traite le résidu dans l'éthanol par un équivalent d'acide fumarique.
On isole 0,87 g de fumarate de (±)cis (méthoxy-4 phényl)-2 acétyloxy-3 (diméthylamino-3 acétyloxy-2 propyl)-5 dihydro-2,3 5H-benzothiazépine-1,5 one-4.
Diastéréoisomère A F = 186°C.

Exemple 2

De manière analogue à l'exemple 1, lorsque l'on traite 3,1 g de (±)cis (méthoxy-4 phényl)-2 hydroxy-3 dihydro-2,3 5H-benzothiazépine-1,5 one-4 avec l'hydrure de sodium puis avec l'épichlorhydrine (1,6 ml) et la pyrrolidine (1,4 ml), on isole 1,4 g de chlorydrate de (±)cis (méthoxy-4 phényl)-2 hydroxy-3 (pyrrolidinyl-3 hydroxy-2 propyl)-5 dihydro-2,3 5H-benzothiazépine-1,5 one-4 sous forme d'un mélange de diastéréoisomères.

F =206°C.

De manière analogue à l'exemple 1, l'acétylation mène au chlorydrate de (±)cis (méthoxy-4 phényl)-2 acétyloxy-3 (pyrrolidinyl-3 acétyloxy-2 propyl)-5 dihydro-2,3 5H-benzothiazépine-1,5 one-4 sous forme de mélange de diastéréoisomères.

F = 241°C.

Exemple 3

Par analogie à l'exemple 1, le traitement de 10 g de (±)cis (méthoxy-4 phényl)-2 hydroxy-3 dihydro-2,3 5H-benzothiazépine-1,5 one-4 par l'hydrure de sodium puis successivement par l'épichlorhydrine (8 ml) et par l'isopropylméthylamine (15 ml) conduit à 5,77 g d'un mélange de diastéréoisomères A et B de (±)cis (méthoxy-4 phényl)-2 hydroxy-3 (N-isopropyl-N-méthyl-amino-3 hydroxy-2 propyl)-5 dihydro-2,3 5H-benzothiazépine-1,5 one-4 sous forme de chlorhydrate.

Par cristallisations successives, on sépare les diastéréoisomères purs.

Diastéréoisomère A F = 181°C

L'acétylation du diastéréoisomère A (1,38 g) par le chlorure d'acétyle permet l'isolement de 1,21 g de chlorhydrate de (±)cis (méthoxy-4 phényl)-2 acétyloxy-3 (N-isopropyl-N-méthyl-amino-3 acétyloxy-2 propyl)-5 dihydro-2,3 5H-benzothiazépine-1,5 one-4.

F = 146°C.

Exemple 4

Par acétylation de 3,1 g d'un mélange de diastéréoisomères du chlorhydrate de (±)cis (méthoxy-4 phényl)-2 hydroxy-3 (N-isopropyl-N-méthyl-amino-3 hydroxy-2 propyl)-5 dihydro-2,3 5H-benzothiazépine-1,5 one-4 dans les conditions décrites précédemment, on obtient 2,9 g de chlorhydrate de (±)cis (méthoxy-4 phényl)-2 acétyloxy-3 (N-isopropyl-N-méthyl-amino-3 acétyloxy-2 propyl)-5 dihydro-2,3 5H-benzothiazépine-1,5 one-4.

F = 152°C.

Exemple 5

On traite 1,5 g de mélange de diastéréoisomères du chlorhydrate de (±)cis (méthoxy-4 phényl)-2 hydroxy-3 (diméthylamino-3 hydroxy-2 propyl)-5 dihydro-2,3 5H-benzothiazépine-1,5 one-4 dans le dichlorométhane et la pyridine, à 0°C par 1 ml de chlorure de pivaloyle. Après isolement et formation du chlorhydrate on recueille 1,6 g de (±)cis (méthoxy-4 phényl)-2 pivaloyloxy-3 (diméthylamino-3 pivaloyloxy-2 propyl)-5 dihydro-2,3 5H-benzothiazépine-1,5 one-4.

F = 118°C.

Exemple 6

Dans un réacteur de 200 ml sous atmosphère inerte, on introduit 0,54 g (0,0133 mole) d'hydrure de sodium à 50% et 55 ml de diméthylsulfoxyde. On agite vigoureusement le mélange durant 1 h à 70°C, puis on le laisse revenir à la température ambiante. On ajoute goutte à goutte 3,93 g (0,013 mole) de (+)cis 2S,3S-(méthoxy-4 phényl)-2 hydroxy-3 dihydro-2,3 5H-benzothiazépine-1,5 one-4 dissoute dans 12 ml de diméthylsulfoxyde. On maintient l'agitation à 30°C durant 1 h puis on ajoute 2,25 ml (0,024 mole) de (+)S-épichlorhydrine (préparée selon la méthode de Baldwin, J. Org. Chem 43, 4876 (1978)), et on continue l'agitation durant 2 h à cette température. On traite le mélange réactionnel par 10 g de diméthylamine dans du diméthylsulfoxyde, on le chauffe à 60°C durant 2 h puis on le laisse au contact durant une nuit.

On verse le mélange réactionnel sur une solution saturée de bicarbonate de sodium glacée, on l'extrait à l'éther, et on le lave à l'eau. Après séchage et évaporation, on isole 4,68 g de (+)cis 2S,3S-(méthoxy-4 phényl)-2 hydroxy-3 (diméthylamino-3 hydroxy-2 propyl)-5 dihydro-2,3 5H-benzothiazépine-1,5 one-4 qu'on acétyle immédiatement par le chlorure d'acétyle dans la pyridine. Après extraction et lavage, on isole 4,5 g de produit brut qu'on traite par l'éthanol chlorhydrique.

Par cristallisation dans l'acétate d'éthyle, on isole 3,55 g de chlorhydrate de (+)cis 2S,3S-(méthoxy-4 phényl)-2 acétyloxy-3 (diméthylamino-3R acétyloxy-2 propyl)-5 dihydro-2,3 5H-benzothiazépine-1,5 one-4.

F = 140-142°C. $[\alpha]_D^{20}$ = +135° (c = 0,1%, $CH_3OH$).

Exemple 7

Dans un réacteur de 150 ml sous atmosphère inerte, on introduit 0,393 g (0,0082 mole) d'hydrure de sodium à 50% et 40 ml de diméthylsulfoxyde. On agite le mélange durant 1 h à 70°C, on le refroidit à 20°C, et on ajoute 2,37 g (0,0079 mole) de (+)cis 2S,3S-(méthoxy-4 phényl)-2 hydroxy-3 dihydro2,3 5H-benzothiazépine-1,5 one-4 dissoute dans 20 ml de diméthylsulfoxyde. On maintient l'agitation à 30°C durant 1 h, on laisse refroidir à 25°C, et on ajoute 2 g (0,0088 mole) de tosylate de (−)R-glycidyle en une seule fois, et on continue l'agitation durant 2 h 30 à cette température. On ajoute 5 g (0,11 mole) de diméthylamine anhydre, on chauffe le mélange à 40°C durant 2 h, et on le laisse sous agitation pendant une nuit sans chauffer.
On verse le mélange sur une solution saturée de bicarbonate de sodium glacée, on l'extrait trois fois à l'éther, on lave la phase organique à l'eau et on la sèche sur sulfate de magnésium. On évapore l'éther et on purifie les 3,2 g de résidu brut par chromatographie sur colonne de silice, en éluant avec un mélange 90/10/2 de dichlorométhane/méthanol/ammoniaque.
On obtient 2 g de base pure qu'on traite avec un équivalent d'acide fumarique et, après recristallisation dans l'éthanol, on isole 1,7 g de fumarate de (+)cis 2S,3S-(méthoxy-4 phényl)-2 hydroxy-3 (diméthylamino-3R hydroxy-2 propyl)-5 dihydro-2,3 5H-benzothiazépine-1,5 one-4.
F = 158°C. $[\alpha]_D^{20}$ = +123° (c = 1%, $CH_3OH$).
Dans un réacteur de 100 ml équipé d'une garde à chlorure de calcium et d'un réfrigérant on introduit 1,62 g (0,004 mole) de ce composé, sous forme de base libre, on ajoute 20 ml d'acide acétique et 20 ml d'anhydride acétique, et on chauffe le mélange à 100°C et sous agitation durant 6 h.
Puis on transfère le mélange dans un évaporateur de 250 ml et on l'évapore sous vide, en ajoutant du toluène pour entraîner les dernières traces d'anhydride acétique. On obtient ainsi 1,7 g de résidu brut dont on prépare le chlorhydrate par traitement dans l'éthanol avec de l'éther chlorhydrique, ce qui donne 1,7 g de chlorhydrate de (+)cis 2S,3S-(méthoxy-4 phényl)-2 acétyloxy-3 (diméthylamino-3R acétyloxy-2 propyl)-5 dihydro-2,3 5H-benzothiazépine-1,5 one-4.
F = 142°C. $[\alpha]_D^{20}$ = +131° (c = 0,1%, $CH_3OH$).

Le tableau de la page suivante illustre les structures et propriétés physiques de quelques composés selon l'invention.

## Tableau

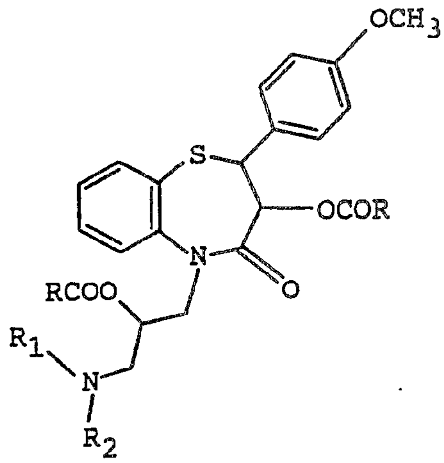

(I)

| Composé n° | R | $R_1$ | $R_2$ | Forme | Sel (*) | F (°C) |
|---|---|---|---|---|---|---|
| 1 (Ex.1) | $CH_3$ | $CH_3$ | $CH_3$ | (±) A+B | 10 | 232 |
| 2 (Ex.1) | $CH_3$ | $CH_3$ | $CH_3$ | (±) B | 10 | 145 |
| 3 (Ex.1) | $CH_3$ | $CH_3$ | $CH_3$ | (±) A | 08 | 186 |
| 4 (Ex.6-7) | $CH_3$ | $CH_3$ | $CH_3$ | (+) A | 10 | 142 |
| 5 (Ex.4) | $CH_3$ | $CH_3$ | $CH(CH_3)_2$ | (±) A+B | 10 | 152 |
| 6 (Ex.3) | $CH_3$ | $CH_3$ | $CH(CH_3)2$ | (±) A | 10 | 146 |
| 7 (Ex.5) | $C(CH_3)_3$ | $CH_3$ | $CH_3$ | (±) A+B | 10 | 118 |
| 8 (Ex.2) | $CH_3$ | $(CH_2)_4$ | | (±) A+B | 10 | 241 |

(*) 08 : fumarate
10 : chlorhydrate

Les composés de l'invention ont été soumis à des essais pharmacologiques montrant leur activité comme antagoniste du calcium.

Le protocole expérimental utilisé est une variante de celui utilisé par Godfraind et Kaba (1969), (Blockade or reversal of the contraction induced by calcium and adrenaline in depolarized arterial smooth muscle, Br. J. Pharmac., 36, 549-560).

Les expériences ont été réalisées sur des tronçons d'aorte thoracique de lapin. Les animaux, des "Fauves de Bourgogne" d'un poids moyen de 1,5 kg, sont sacrifiés par dislocation cervicale et exsanguination. L'aorte thoracique est rapidement prélevée et placée dans un milieu de Krebs bicarbonaté oxygéné (95% $O_2$ + 5% $CO_2$).

Des tronçons d'aorte de 1 cm de long environ sont préparés et installés dans des cuves à organes de 20 ml contenant de la solution de Krebs bicarbonatée oxygénée (pH 7,4) à 37°C. Deux crochets métalliques en "U" de la longueur des tronçons sont introduits dans la lumière de ceux-ci. L'un des crochets est fixé à la base de la cuve. L'autre, relié à une jauge de contrainte isométrique (Grass FT03), permet l'enregistrement, par l'intermédiaire d'un préamplificateur continu (Grass 7P1), des réponses contractiles des tronçons d'aorte sur un oscillographe à encre (Grass 79B). Cette méthode présente, par rapport aux préparations en spirale ou en anneaux, l'avantage de mieux respecter l'intégrité structurale des vaisseaux et de n'enregistrer que la composante radiale des réponses contractiles qui représente le phénomène intéressant du point de vue fonctionnel (régulation de la pression artérielle). Une tension initiale de 4 g est imposée aux préparations.

De la phénoxybenzamine (1 µM) et du propranolol (1 µM) sont ajoutés aux différents milieux de Krebs afin de supprimer les réponses contractiles liées à l'activation des récepteurs α-et β-adrénergiques vasculaires.

Après une heure de stabilisation dans le milieu de Krebs bicarbonaté, la tension imposée aux aortes est ramenée à 2 g. Après une période d'attente de 30 minutes, les préparations sont incubées pendant une dizaine de minutes dans une solution de Krebs bicarbonatée sans calcium en présence d'EDTA (200 µM) et de propranolol (1 µM). Cette solution est alors remplacée par un milieu de Krebs dépolarisant (riche en potassium) sans calcium et contenant du propranolol (1 µM). Après 5 minutes, une concentration unique de 1 mM de calcium est ajoutée à cette solution et une période de stabilisation de 30 minutes est respectée qui permet aux préparations d'atteindre une contraction stable.

Ensuite, des doses cumulatives des composés à tester sont administrées toutes les 30 minutes (temps généralement nécessaire pour l'obtention d'un palier) jusqu'à disparition totale de la contraction provoquée par 1 mM de calcium ou bien jusqu'à la concentration maximale de 30 µM de produit. En fin d'expérience, une concentration supramaximale de papavérine (300 µM) est administrée afin de déterminer la décontraction maximale possible de chaque préparation.

Les valeurs absolues (en grammes) de la contraction initiale après 1 mM de $CaCl_2$) et de la contraction après les différentes concentrations cumulatives de composés vasodilateurs sont obtenues, pour chaque préparation, par différence avec la contraction minimale observée 30 minutes après l'addition finale de 300 µM de papavérine. Le pourcentage de diminution de la contraction, par rapport a la contraction provoquée par 1 mM de calcium, est calculé pour chaque dose de composé et chaque préparation et ce pourcentage individuel de décontraction est moyenné $\overline{X} \pm$ S.E.M. Les valeurs moyennes obtenues(pondérées par l'inverse de l'erreur standard à la moyenne), sont analysées à l'aide d'un modèle mathématique de courbe sigmoïde. On calcule la concentration molaire provoquant 50% de décontraction de la réponse au calcium ($CE_{50}$), ou bien son antilogarithme ($PCE_{50}$).

Pour les composés de l'invnetion le $PCE_{50}$ est de l'ordre de 5,8 à 6.

Les résultats des essais montrent que les composés de l'invention peuvent être utilisés pour le traitement de toutes maladies où des antagonistes du calcium peuvent être utilisés, telles que angine de poitrine, dysrythmie d'origine supraventriculaire, hypertension, cardiomyopathie, protection myocardiaque de patients à risque d'infarctus ou ayant subi un infarctus, arrêt du coeur, accidents cérébrovasculaires, manie, migraines.

Les composés de l'invention peuvent être présentés sous toute forme appropriée à l'administration orale ou parentérale, en association avec tout excipient approprié, par exemple sous forme de comprimés, gélules, capsules, solutions buvables ou injectables.

La posologie journalière peut aller de 10 à 400 mg par voie orale et de 1 à 50 mg par voie parentérale.

## Revendications

### Revendications pour les Etats Contractants : BE, CH, DE, FR, GB, IT, LI, LU, NL, SE.

1. Composé, sous forme d'isomère optique pur, de diastéréoisomère ou de mélanges, caractérisé en ce qu'il répond à la formule générale (I)

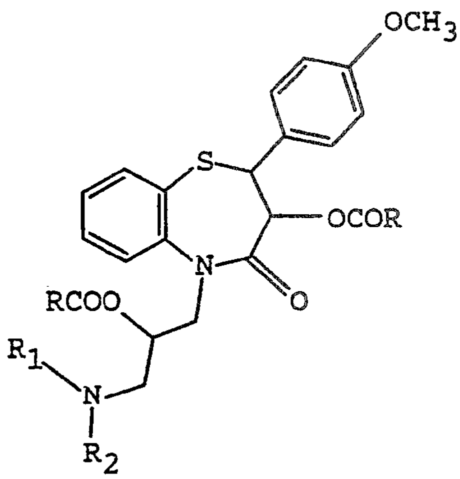

(I)

dans laquelle

R représente l'hydrogène ou un groupe alkyle en $C_1$-$C_4$ linéaire ou ramifié, et

$R_1$ et $R_2$, séparément, représentent chacun un groupe alkyle en $C_1$-$C_4$ linéaire ou ramifié, ou bien

$R_1$ et $R_2$, ensemble, représentent un groupe tétraméthylène ou pentaméthylène,

ainsi que ses sels d'addition à des acides acceptables en pharmacologie.

2. Composé selon la revendication 1, caractérisé en ce que, dans la formule (I), R, $R_1$ et $R_2$ représentent chacun un groupe méthyle.

3. Composé selon la revendication 1, en l'espèce la (+)cis 2S,3S-(méthoxy-4 phényl)-2 acétyloxy-3 (diméthylamino-3R acétyloxy-2 propyl)-5 dihydro-2,3 5H-benzothiazépine-1,5 one-4.

4. Procédé de préparation d'un composé selon la revendication 1, caractérisé en ce qu'on traite une benzothiazépinone de formule (II)

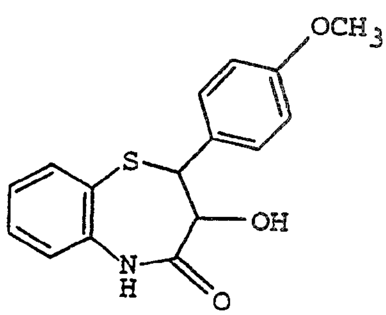

(II)

par l'hydrure de sodium, puis on fait réagir le composé obtenu avec un dérivé glycidylique de formule

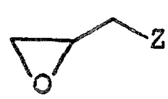

dans laquelle Z représente un groupe chloro ou tosyloxy, puis avec une amine de formule $HNR_1R_2$, $R_1$ et $R_2$ étant tels que définis dans la revendication 1, et enfin on soumet le composé obtenu, de formule (III)

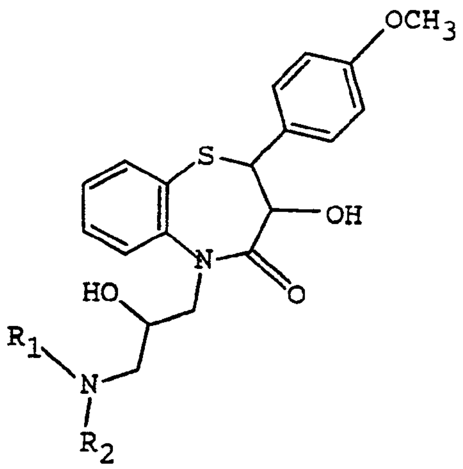

(III)

a une acylation au moyen d'un halogénure d'acyle de formule RCOX, R étant tel que défini dans la revendication 1.

5. Composé nécessaire comme intermédiaire dans le procédé selon la revendication 4, caractérisé en ce qu'il répond à la formule (III)

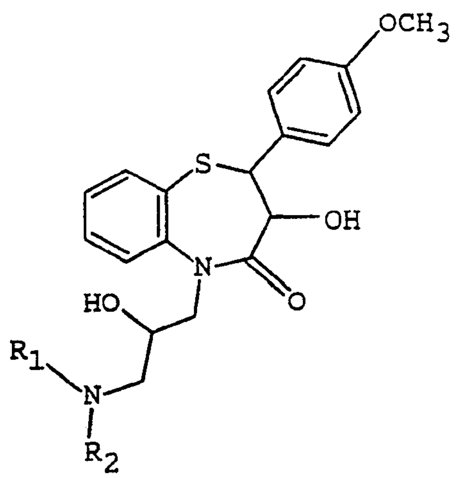

(III)

dans laquelle $R_1$ et $R_2$ sont tels que définis dans la revendication 1.

6. Composé selon la revendication 5, en l'espèce la (+)cis 2S,3S-(méthoxy-4 phényl)-2 hydroxy-3 (diméthylamino-3R hydroxy-2 propyl)-5 dihydro-2,3 5H-benzothiazépine-1,5 one-4.

7. Médicament, caractérisé en ce qu'il consiste en un composé selon l'une des revendications 1 à 3.

8. Composition pharmaceutique, caractérisée en ce qu'elle contient un composé selon l'une des revendications 1 à 3, associé à un excipient approprié.

**Revendications pour les Etats Contractants : AT, ES, GR.**

1. Procédé de préparation d'un composé répondant à la formule générale (I)

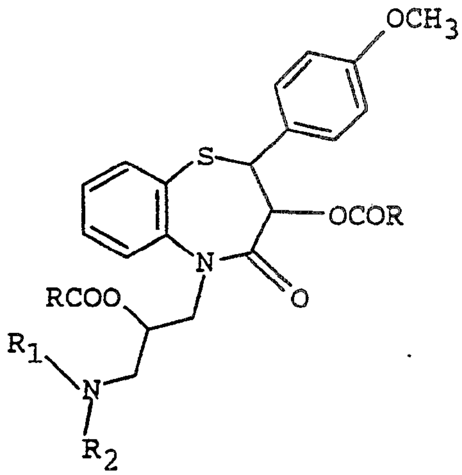

(I)

dans laquelle

R représente l'hydrogène ou un groupe alkyle en $C_1$-$C_4$ linéaire ou ramifié, et

$R_1$ et $R_2$, séparément, représentent chacun un groupe alkyle en $C_1$-$C_4$ linéaire ou ramifié, ou bien

$R_1$ et $R_2$, ensemble, représentent un groupe tétraméthylène ou pentaméthylène,

procédé caractérisé en ce qu'on traite une benzothiazépinone de formule (II)

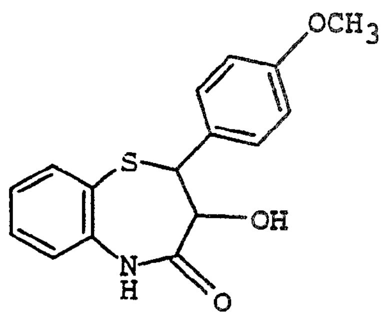

(II)

par l'hydrure de sodium, puis on fait réagir le composé obtenu avec un dérivé glycidylique de formule

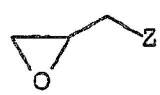

dans laquelle Z représente un groupe chloro ou tosyloxy, puis avec une amine de formule $HNR_1R_2$, $R_1$ et $R_2$ étant tels que définis ci-dessus, et enfin on soumet le composé obtenu, de formule (III)

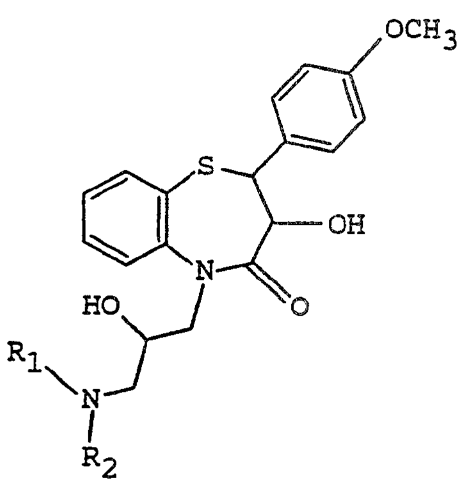

(III)

à une acylation au moyen d'un halogénure d'acyle de formule RCOX, R étant tel que défini ci-dessus.

2. Procédé selon la revendication 1, caractérisé en ce que le composé de formule (II) est la (+)cis 2S,3S-(méthoxy-4 phényl)-2 hydroxy-3 dihydro-2,3 5H-benzothiazépine-1,5 one-4 et le dérivé glycidylique est la (+)S-épichlorhydrine ou le tosylate de (–)R-glycidyle.

## Ansprüche

### Patentansprüche für die Vertragsstaaten : BE, CH, DE, FR, GB, IT, LI, LU, NL, SE

1. Verbindung in Form des reinen optischen Isomeren, des Diastereomeren oder von Mischungen, dadurch gekennzeichnet, daß sie der allgemeinen Formel (I)

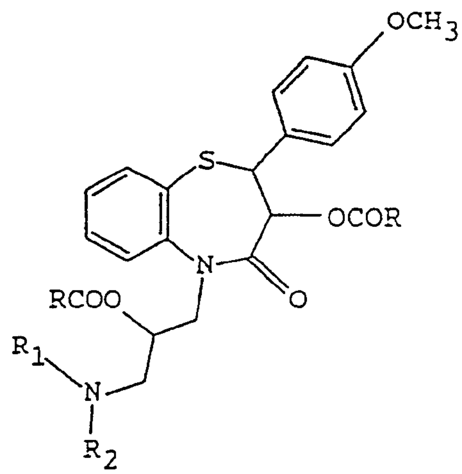

(I)

entspricht, in welcher R für Wasserstoff oder eine lineare oder verzweigte $C_1$-$C_4$-Alkylgruppe steht und $R_1$ und $R_2$ unabhängig voneinander jeweils eine lineare oder verzweigte $C_1$-$C_4$-Alkylgruppe bedeuten oder $R_1$ und $R_2$ gemeinsam für eine Tetramethylen- oder Pentamethylengruppe stehen, sowie deren Säureadditionssalze mit pharmakologisch verwendbaren Säuren.

2. Verbindung nach Anspruch 1, dadurch gekennzeichnet, daß in Formel (I) R, $R_1$ und $R_2$ jeweils für eine Methylgruppe stehen.

3. Verbindung nach Anspruch 1 in Form des (+)cis-2S,3S-2-(4-methoxyphenyl)-3-acetyloxy-5-(3R-dimethylamino-2-acetyloxypropyl)-2,3-dihydro-1,5-5H-benzothiazepin-4-ons.

4. Verfahren zur Herstellung einer Verbindung nach Anspruch 1, dadurch gekennzeichnet, daß man ein Benzothiazepinon der Formel (II)

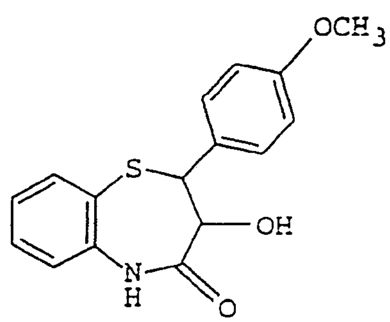

(II)

mit Natriumhydrid behandelt, dann die erhaltene Verbindung mit einem Glycidylderivat der Formel

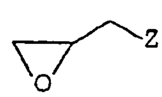

in welcher Z für eine Chlor- oder Tosyloxygruppe steht, zur Reaktion bringt, dann mit einem Amin der Formel

$HNR_1R_2$, in welcher $R_1$ und $R_2$ die in Anspruch 1 genannte Bedeutung haben, umsetzt und schließlich die erhaltene Verbindung der Formel (III)

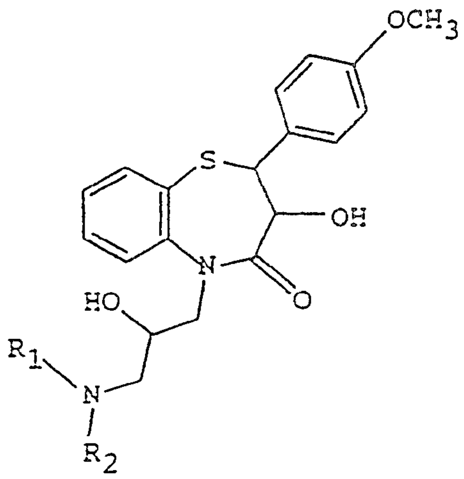

(III)

einer Acylierung mit Hilfe eines Acylhalogenids der Formel RCOX, in der R die in Anspruch 1 genannte Bedeutung hat, unterwirft.

5. Verbindung, die als Zwischenprodukt im Verfahren nach Anspruch 4 erforderlich ist, dadurch gekennzeichnet, daß sie der Formel (III)

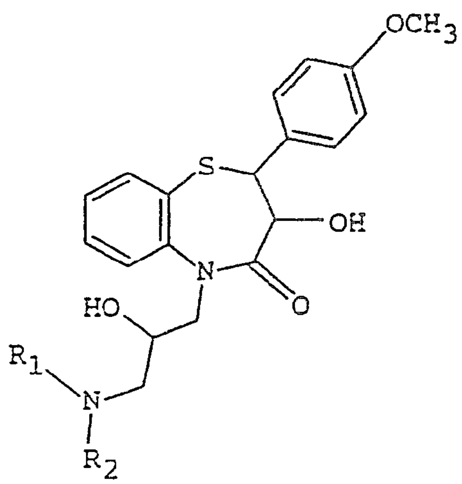

(III)

entspricht, in welcher $R_1$ und $R_2$ die in Anspruch 1 genannte Bedeutung haben.

6. Verbindung nach Anspruch 5 in Form des (+)cis-2S,3S,-2-(4-Methoxyphenyl)-3-hydroxy-5-(3R-dimethylamino-2-hydroxypropyl)-2,3-dihydro-1,5-5H-Benzothiazepin-4-ons.

7. Arzneimittel, dadurch gekennzeichnet, daß es aus einer Verbindung nach einem der Ansprüche 1 bis 3 besteht.

8. Pharmazeutische Zusammensetzung, dadurch gekennzeichnet, daß sie eine Verbindung nach einem der Ansprüche 1 bis 3 in Kombination mit einem geeigneten Träger enthält.

**Patentansprüche für die Vertragsstaaten : AT, ES, GR**

1. Verfahren zur Herstellung einer Verbindung der allgemeinen Formel (I)

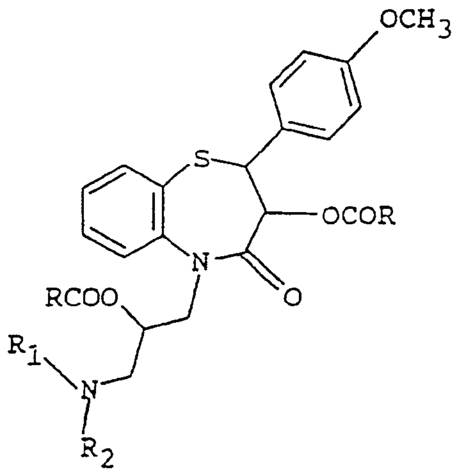

(I)

in welcher R für Wasserstoff oder eine lineare oder verzweigte $C_1$-$C_4$-Alkylgruppe steht und $R_1$ und $R_2$ unabhängig voneinander jeweils eine lineare oder verzweigte $C_1$-$C_4$-Alkylgruppe bedeuten oder $R_1$ und $R_2$ gemeinsam für eine Tetramethylen- oder Pentamethylengruppe stehen, dadurch gekennzeichnet, daß man ein Benzothiazepinon der Formel (II)

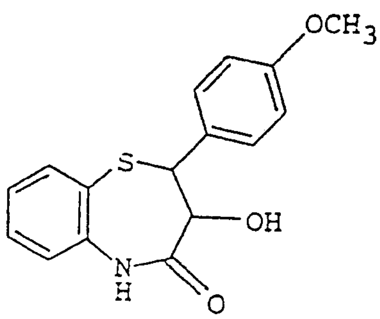

(II)

mit Natriumhydrid behandelt, dann die erhaltene Verbindung mit einem Glycidylderivat der Formel

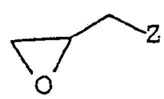

in welcher Z für eine Chlor- oder Tosyloxygruppe steht, zur Reaktion bringt, dann mit einem Amin der Formel $HNR_1R_2$, in welcher $R_1$ und $R_2$ die oben genannte Bedeutung haben, umsetzt und schließlich die erhaltene Verbindung der Formel (III)

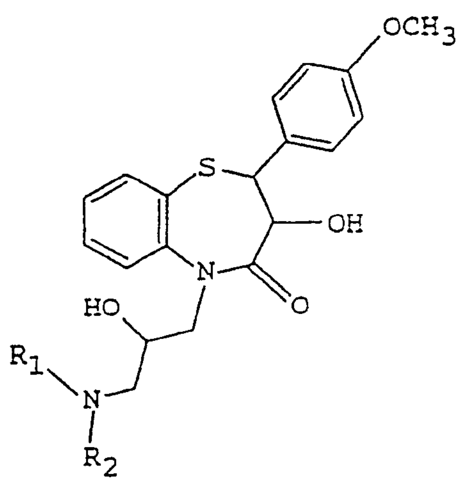

(III)

einer Acylierung mit Hilfe eines Acylhalogenids der Formel RCOX, in der R die oben genannte Bedeutung hat, unterwirft.

2. Verfahren nach Anspruch 1, dadurch gekennzeichnet, daß die Verbindung der Formel (II) das (+)cis-2S,3S-2-(4-Methoxyphenyl)-3-hydroxy-2,3-dihydro-1,5-5H-benzothiazepin-4-on und das Glycidylderivat das (+)S-Epichlorhydrin oder das (–)R-Glycidyltosylat ist.

## Claims

### Claims for the Contracting States : BE, CH, DE, FR, GB, IT, LI, LU, NL, SE.

1. Compound, in the form of pure optical isomer, diastereoisomer or mixtures, characterised in that it corresponds to the general formula (I)

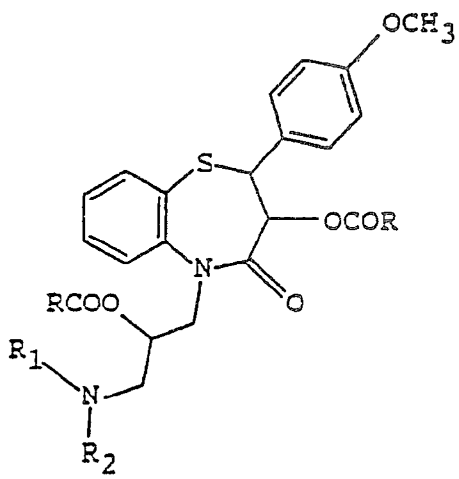

(I)

in which

R denotes hydrogen or a linear or branched $C_1$-$C_4$ alkyl group, and

$R_1$ and $R_2$, separately, each denote a linear or branched $C_1$-$C_4$ alkyl group, or alternatively

$R_1$ and $R_2$ together denote a tetramethylene or pentamethylene group,

as well as its addition salts with acids which are pharmacologically acceptable.

2. Compound according to Claim 1, characterised in that, in the formula (I), R, $R_1$ and $R_2$ each denote a methyl group.

3. Compound according to Claim 1, namely (+)-cis(2S, -3S)-2-(4-methoxyphenyl)-3-acetyloxy-5-[(3R)-3-dimethyl-amino-2-acetyloxypropyl]-2,3-dihydro-5H-1,5-benzothiazepin-4-one.

4. Process for preparing a compound according to Claim 1, characterised in that a benzothiazepinone of formula (II)

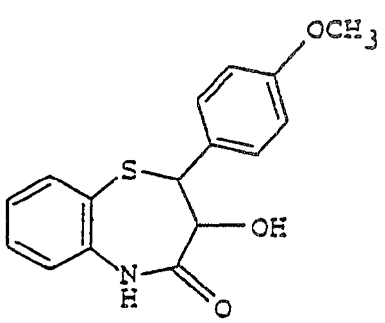

(II)

is treated with sodium hydride, the compound obtained is then reacted with a glycidyl derivative of formula

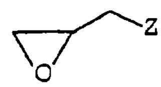

in which Z denotes a chloro or tosyloxy group, and then with an amine of formula $HNR_1R_2$, $R_1$ and $R_2$ being as defined in Claim 1, and finally the compound obtained, of formula (III)

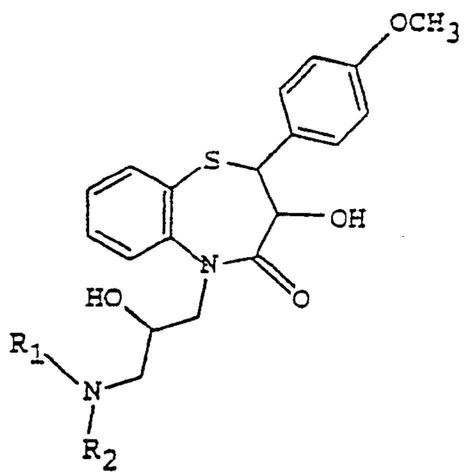

(III)

is subjected to a acylation by means of an acyl halide of formula RCOX, R being as defined in Claim 1.

5. Compound needed as an intermediate in the process according to Claim 4, characterised in that it corresponds to the formula (III)

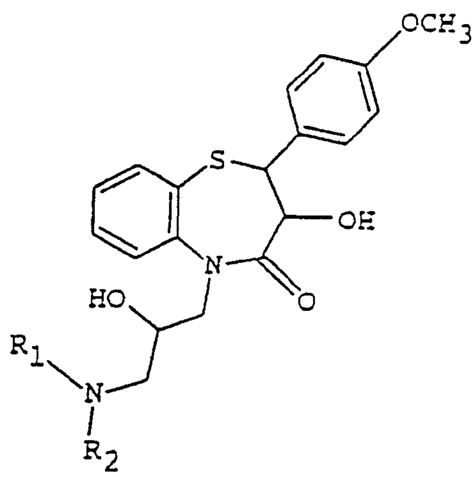

(III)

in which $R_1$ and $R_2$ are as defined in Claim 1.

6. Compound according to Claim 5, namely (+)-cis-(2S,3S)-2-(4-methoxyphenyl)-3-hydroxy-5-[(3R)-3-dimethyl-amino-2-hydroxypropyl]-2,3-dihydro-5H-1,5-benzothiazepin-4-one.

7. Medicinal product, characterised in that it consists of a compound according to one of Claims 1 to 3.

8. Pharmaceutical composition, characterised in that it contains a compound according to one of Claims 1 to 3, combined with a suitable excipient.

**Claims for the Contracting States : AT, ES, GR.**

1. Process for preparing a compound corresponding to the general formula (I)

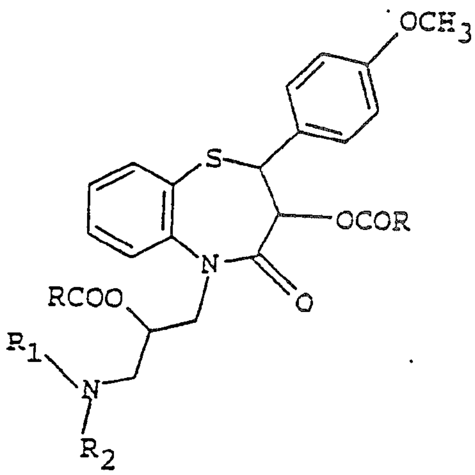

(I)

in which

R denotes hydrogen or a linear or branched $C_1$-$C_4$ alkyl group, and

$R_1$ and $R_2$, separately, each denote a linear or branched $C_1$-$C_4$ alkyl group, or alternatively $R_1$ and $R_2$ together denote a tetramethylene or pentamethylene group,

which process is characterised in that a benzothiazepinone of formula (II)

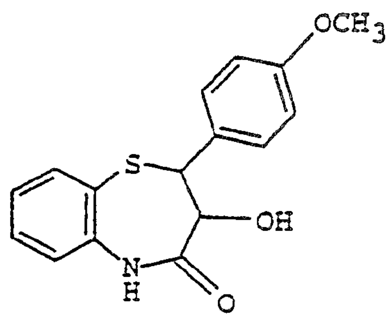

(II)

is treated with sodium hydride, the compound obtained is then reacted with a glycidyl derivative of formula

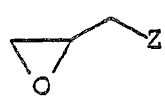

in which Z denotes a chloro or tosyloxy group, and then with an amine of formula $HNR_1R_2$, $R_1$ and $R_2$ being as defined above, and finally the compound obtained, of formula (III)

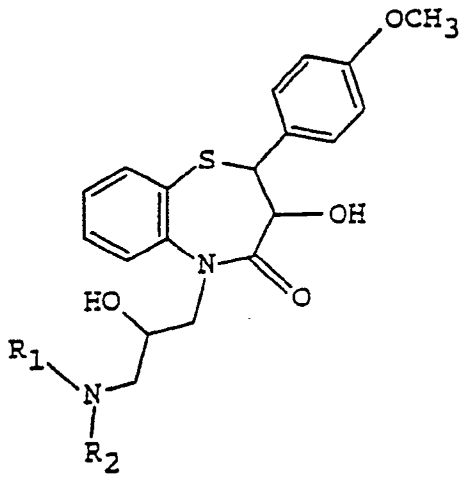

(III)

is subjected to an acylation by means of an acyl halide of formula RCOX, R being as defined above.

2. Process according to Claim 1, characterised in that the compound of formula (II) is (+)-cis-(2S,3S)-2-(4-methoxyphenyl)-3-hydroxy-2,3-dihydro-5H-1,5-benzothiazepin-4-one and the glycidyl derivative is (+)-(S)-epichlorohydrin or (–)-(R)-glycidyl tosylate.